(19) Europäisches Patentamt
European Patent Office
Office européen des brevets

(11) Veröffentlichungsnummer: **0 002 207**
**B1**

# (12) EUROPÄISCHE PATENTSCHRIFT

(45) Veröffentlichungstag der Patentschrift: **02.09.81**

(51) Int. Cl.³: **C 07 C 49/587,** C 07 C 45/00, C 07 C 61/16 // A01N53/00

(21) Anmeldenummer: **78101378.4**

(22) Anmeldetag: **16.11.78**

## (54) Dichlorvinylcyclobutanone, Verfahren zu deren Herstellung und ihre Verwendung als Zwischenprodukte für die Herstellung von Schädlingsbekämpfungsmitteln.

(30) Priorität: **24.11.77 CH 14406/77**
**26.10.78 CH 11076/78**

(43) Veröffentlichungstag der Anmeldung:
**13.06.79 Patentblatt 79/12**

(45) Bekanntmachung des Hinweises auf die Patenterteilung:
**02.09.81 Patentblatt 81/35**

(84) Benannte Vertragsstaaten:
**BE CH DE FR GB NL**

(56) Entgegenhaltungen:
**DE-A-2 539 048**
**DE-A-2 813 336**

(73) Patentinhaber: **CIBA-GEIGY AG, Patentabteilung Postfach, CH-4002 Basel (CH)**

(72) Erfinder: **Martin, Pierre, Dr., Meisenweg 38, CH-4310 Rheinfelden (CH)**
Erfinder: **Bellus, Daniel, Dr., Unterm Schellenberg 81, CH-4125 Riehen (CH)**

**EP 0 002 207 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Dichlorvinylcyclobutanone, Verfahren zu deren Herstellung und ihre Verwendung als Zwischenprodukte für die Herstellung von Schädlingsbekämpfungsmitteln

Die vorliegende Erfindung betrifft Dichlorvinylcyclobutanone und ein Verfahren zu deren Herstellung. Die Dichlorvinylcyclobutanone stellen Zwischenprodukte für die Herstellung von Schädlingsbekämpfungsmitteln dar.

Die Dichlorvinylcyclobutanone entsprechen der Formel

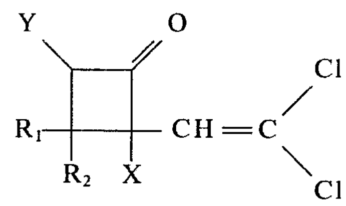

worin eines von $R_1$ und $R_2$ Methyl und das andere Wasserstoff oder Methyl oder $R_1$ und $R_2$ zusammen mit 2—4 C-Atomen darstellen, X Chlor und Y Wasserstoff oder X Wasserstoff und Y Chlor bedeuten.

Die neuen Dichlorvinylcyclobutanone der Formel I können nach dem erfindungsgemäßen Verfahren dadurch hergestellt werden, daß man eine Verbindung der Formel

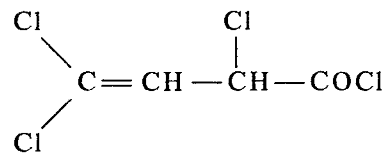

(II)

in Gegenwart einer organischen Base mit einer Verbindung der Formel

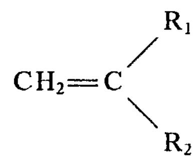

(III)

zu einer Verbindung der Formel

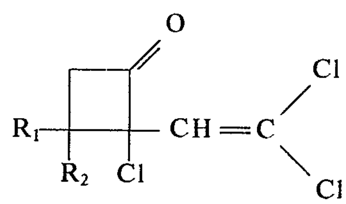

(Ia)

umsetzt, wobei $R_1$ und $R_2$ die unter Formel I angegebene Bedeutung haben, und die Verbindung der Formel Ia in Gegenwart eines Katalysators gegebenenfalls zu einer Verbindung der Formel I umlagert, worin X Wasserstoff und Y Chlor bedeuten.

Bevorzugt sind Verbindungen der Formel I, worin $R_1$ und $R_2$ zusammen Äthylen darstellen und insbesondere Verbindungen der Formel I, worin $R_1$ und $R_2$ je Methyl darstellen.

Bei der Umsetzung des Säurechlorids der Formel II in Gegenwart der organischen Base wird intermediär ein Keten der Formel

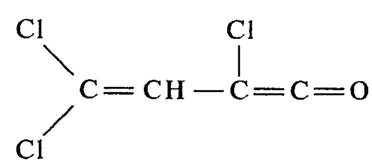

(IV)

gebildet. Das Keten der Formel IV ist neu.

Die Umsetzung des Säurechlorids der Formel II mit dem Olefin der Formel III wird zweckmäßig in Gegenwart eines inerten organischen Lösungsmittels vorgenommen. Als solche eignen sich z. B. gegebenenfalls halogenierte aromatische oder aliphatische Kohlenwasserstoffe, wie Benzol, Toluol, Xylole, Chlorbenzol, Dichlor- und Trichlorbenzole, n-Pentan, n-Hexan, n-Octan, Methylenchlorid, Chloroform, Tetrachlormethan, 1,1,2,2-Tetrachloräthan und Trichloräthylen; cycloaliphatische Kohlenwasserstoffe, wie Cyclopentan oder Cyclohexan; aliphatische oder cycloaliphatische Ketone,

wie Aceton, Methyläthylketon, Cyclopentanon und Cyclohexanon; aliphatische und cyclische Äther, wie Diäthyläther, Tetrahydrofuran, Tetrahydropyran und Dioxan, sowie Nitrile von gesättigten aliphatischen Monocarbonsäuren mit insgesamt 1—6 Kohlenstoffatomen, wie Acetonitril, Propionitril, 3-Methoxypropionitril und Butyronitril.

Bevorzugt für diese Verfahrensstufe sind aliphatische, cycloaliphatische und aromatische Kohlenwasserstoffe, vor allem Alkane mit 5—8 C-Atomen, Benzol und Toluol, ganz besonders jedoch n-Hexan und Cyclohexan.

Die Umsetzung kann jedoch auch ohne Zusatz eines inerten organischen Lösungsmittels vorgenommen werden, z. B. in überschüssigem Olefin der Formel III.

Geeignete organische Basen sind zum Beispiel tertiäre Amine, vor allem Trialkylamine mit je 1—4, insbesondere 2—4 Kohlenstoffatomen in den Alkylteilen; cyclische Amine, wie Pyridin, Chinolin, N-Alkyl-Pyrrolidine, N-Alkyl-Piperidine, N,N'-Dialkyl-Piperazine und N-Alkyl-Morpholine oder Dialkylaniline mit je 1 oder 2 Kohlenstoffatomen in den Alkylteilen, wie N-Methylpyrrolidin, N-Äthylpiperidin, N,N'-Dimethylpiperazin, N-Äthylmorpholin und Dimethylanilin, sowie bicyclische Amidine, wie 1,5-Diazabicyclo-[5.4.0]undec-5-en und 1,5-Diazabicyclo [4.3.0]non-5-en, und bicyclische Aminoverbindungen, wie 1,4-Diazabicyclo[2.2.2]octan.

Bevorzugte Basen sind Trialkylamine mit je 1—4 C-Atomen in den Alkylteilen, besonders Triäthylamin, und Pyridin.

Die organische Base wird in mindestens äquimolarer Menge zum Säurechlorid der Formel II oder aber in einem schwachen Überschuß eingesetzt.

Die Olefine der Formel II werden in mindestens äquimolarer Menge, bezogen auf das Säurechlorid der Formel II bzw. das in situ gebildete Keten der Formel IV, verwendet. Im allgemeinen ist es aber zweckmäßig, einen Überschuß an Olefin zu verwenden, wobei das Olefin, wie schon erwähnt, auch als Lösungsmittel dienen kann.

Die Reaktionstemperaturen können innerhalb weiter Grenzen variieren. Sie liegen im allgemeinen zwischen etwa 0 und 200° C, bevorzugt zwischen etwa 20 und 150° C.

Die Umsetzung kann zweckmäßig so vorgenommen werden, daß man die Verbindungen der Formel II und III vorlegt und dann die organische Base zutropft, oder aber indem man das Olefin der Formel III vorlegt und getrennt, aber gleichzeitig das Säurechlorid und die organische Base zutropft. Schließlich ist es auch möglich, organische Base und Olefin vorzulegen und dann das Säurechlorid zuzutropfen.

Mit leichtflüchtigen Olefinen der Formel III kann die Umsetzung auch unter Druck vorgenommen werden.

Nach Beendigung der Umsetzung können die Verbindungen der Formel Ia gewünschtenfalls auf übliche Weise isoliert und gegebenenfalls gereinigt werden, beispielsweise durch Filtrieren und Umkristallisieren aus geeigneten organischen Lösungsmitteln, wie n-Hexan.

Für die Umlagerung der Verbindungen der Formel Ia in Verbindungen der Formel I mit X=Wasserstoff und Y=Chlor können als Katalysatoren Säuren, Basen oder quaternäre Ammoniumhalogenide eingesetzt werden.

Die definitionsgemäße Umlagerung ist völlig unerwartet und weder bei $\alpha$-monohalogenierten Cyclobutanonen im speziellen, noch bei $\alpha$-monohalogenierten Ringketonen im allgemeinen bekannt.

Als basische Katalysatoren kommen organische Basen in Betracht, wie primäre, sekundäre und insbesondere tertiäre Amine der Formel

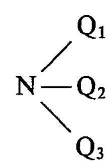

worin $Q_1$ Alkyl mit 1—8 C-Atomen, Cycloalkyl mit 5 oder 6 C-Atomen, Benzyl oder Phenyl und $Q_2$ und $Q_3$ unabhängig voneinander Wasserstoff oder Alkyl mit 1—8 C-Atomen bedeuten, beispielsweise Triäthylamin, Tri-n-butylamin, Tri-isopentylamin, Tri-n-octylamin, N,N-Dimethyl-cyclohexylamin, N,N-Dimethyl-benzylamin, N,N-Dimethyl-2-äthylhexylamin, N,N-Diäthylanilin; ferner cyclische Amine, wie Pyridin, Chinolin, Lutidin, N-Alkylmorpholine, wie N-Methylmorpholin, N-Alkylpiperidine, wie N-Methyl- und N-Äthylpiperidin, N-Alkylpyrrolidine, wie N-Methyl- und N-Äthylpyrrolidin; Diamine, wie N,N,N',N'-Tetramethyläthylendiamin, N,N,N',N'-Tetramethyl-1,3-diaminobutan, N,N'-Dialkylpiperazine, wie N,N'-Dimethylpiperazin; bicyclische Diamine, wie 1,4-Diaza-bicyclo[2.2.2]-octan und bicyclische Amidine, wie 1,5-Diazabicyclo[5.4.0]undec-5-en und 1,5-Diazabicyclo[4.3.0]non-5-en, und schließlich polymere basische Verbindungen, wie p-Dimethylaminomethylpolystyrol und Phosphine, speziell Trialkylphosphine, wie Tributylphosphin.

Als saure Katalysatoren können z. B. anorganische oder organische Protonensäuren verwendet werden. Beispiele geeigneter anorganischer Protonensäuren sind Halogenwasserstoffsäuren, wie HCl, HBr, HF und HJ, Salpetersäure, Phosphorsäure und Schwefelsäure.

Als organische Protonensäuren kommen z. B. in Betracht: Sulfinsäuren, wie Benzolsulfinsäure; aliphatische und gegebenenfalls substituierte aromatische Sulfonsäuren, wie Methansulfonsäure,

Benzolsulfonsäure, p-Toluolsulfonsäure, Naphthalinsulfonsäure, Naphthalin-1,5-disulfonsäuren; aliphatische Monocarbonsäuren mit vorzugsweise 1—18 C-Atomen, wie Ameisensäure, Essigsäure, Propionsäure, Buttersäure, Laurinsäure, Palmitinsäure, Stearinsäure; halogenhaltige Monocarbonsäuren, wie Chloressigsäure, Dichloressigsäure, Trichloressigsäure und Trifluoressigsäure; aliphatische Dicarbonsäuren mit vorzugsweise 2—12 Kohlenstoffatomen, wie Malonsäure, Bernsteinsäure, Adipinsäure, Sebacinsäure; gegebenenfalls substituierte aromatische Mono- oder Dicarbonsäuren, wie Benzoesäure, Toluylsäure, Naphthoesäure, Phthalsäure und Terphthalsäure; aliphatische und aromatische Phosphon- und Phosphinsäuren, wie Methyl-, Benzyl- oder Phenylphosphonsäure oder Dimethyl- und Diäthylphosphonsäure oder Diäthyl- und Benzolphosphinsäure.

Werden Säuren oder Basen im Überschuß eingesetzt, so können sie auch als Lösungsmittel dienen.

Ferner können Salze von Protonensäuren, besonders Halogenwasserstoffsäuren, mit Ammoniak oder einer Stickstoff enthaltenden organischen Base sowie quaternäre Ammoniumhalogenide eingesetzt werden. Als Stickstoff enthaltende organische Basen eignen sich aliphatische, cycloaliphatische, araliphatische und aromatische primäre, sekundäre und tertiäre Amine sowie heterocyclische Stickstoffbasen. Als Beispiele seien genannt: primäre aliphatische Amine mit bis zu 12 C-Atomen, wie Methylamin, Äthylamin, n-Butylamin, n-Octylamin, n-Dodecylamin, Hexamethylendiamin, Cyclohexylamin, Benzylamin; sekundäre aliphatische Amine mit bis zu 12 C-Atomen, wie Dimethylamin, Diäthylamin, Di-n-propylamin, Dicyclohexylamin, Pyrrolidin, Piperidin, Piperazin, Morpholin; tertiäre aliphatische Amine, besonders Trialkylamine mit je 1—4 C-Atomen in den Alkylteilen, wie Triäthylamin, Tri-n-butylamin, N-Methylpyrrolidin, N-Methylmorpholin, 1,4-Diazabicyclo[2.2.2]octan; Chinuclidin; gegebenenfalls substituierte primäre, sekundäre und tertiäre aromatische Amine, wie Anilin, Toluidin, Naphthylamin, N-Methylanilin, Diphenylamin und N,N-Diäthylanilin; ferner Pyridin, Picolin, Indolin und Chinolin.

Bevorzugt sind Salze der Formel

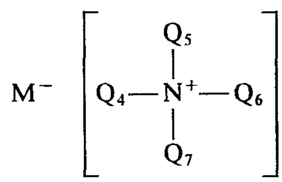

worin

M Fluor, Brom oder Jod, insbesondere Chlor,
$Q_4$ Wasserstoff, Alkyl mit 1—18 C-Atomen, Cyclohexyl, Benzyl, Phenyl oder Naphthyl und
$Q_5$, $Q_6$ und $Q_7$ unabhängig voneinander Wasserstoff oder Alkyl mit 1—18 C-Atomen bedeuten,

sowie N-Alkyl-Pyridiniumhalogenide mit 1—18 C-Atomen im Alkyl, besonders die entsprechenden Chloride.

Beispiele derartiger Salze sind: Ammoniumchlorid, Ammoniumbromid, Methylaminhydrochlorid, Cyclohexylaminhydrochlorid, Anilinhydrochlorid, Dimethylaminhydrochlorid, Di-isobutylaminohydrochlorid, Triäthylaminhydrochlorid, Triäthylaminhydrobromid, Tri-n-octylaminhydrochlorid, Benzyldimethylaminhydrochlorid, Tetramethyl-, Tetraäthyl-, Tetra-n-propyl-, Tetra-n-butylammoniumchlorid, -bromid und -jodid, Trimethyl-hexadecylammoniumchlorid, Benzyldimethylhexadecylammoniumchlorid, Benzyldimethyltetradecylammoniumchlorid, Benzyl-trimethyl-, -triäthyl- und -tri-n-butylammoniumchlorid, n-Butyl-tri-n-propylammoniumbromid, Octadecyltrimethylammoniumbromid, Phenyltrimethylammoniumbromid oder -chlorid, Hexadecylpyridiniumbromid und -chlorid.

Als zusätzliche Co-Katalysatoren kann man Alkalimetallhalogenide, wie Kaliumjodid, Natriumjodid, Lithiumjodid, Kaliumbromid, Natriumbromid, Lithiumbromid, Kaliumchlorid, Natriumchlorid, Lithiumchlorid, Kaliumfluorid, Natriumfluorid und Lithiumfluorid verwenden.

Diese Co-Katalysatoren katalysieren die Reaktion auch in Abwesenheit obiger Ammoniumsalze, jedoch sind dann Zusätze offenkettiger oder makrocyclischer Polyäther (Kronenäther) für den raschen Ablauf von Vorteil. Beispiele solcher Kronenäther sind: 15-Crown-5, 18-Crown-6, Dibenzo-18-crown-6, Dicyclohexyl-18-crown-6, 5,6,14,15-Dibenzo-7,13-diaza-1,4-dioxa-cyclopentadeca-5,14-dien.

Die Menge des eingesetzten Katalysators kann innerhalb breiter Grenzen variieren. In manchen Fällen gnügt es, wenn der Katalysator in Spuren vorliegt. Im allgemeinen wird jedoch der Katalysator bevorzugt in einer Menge von etwa 0,1 bis 15 Gewichtsprozent, bezogen auf die Verbindung der Formel Ia, eingesetzt.

Die Umlagerung kann sowohl in der Schmelze als auch in einem inerten organischen Lösungsmittel vorgenommen werden. Die Reaktionstemperaturen für die Umlagerung in der Schmelze liegen im allgemeinen etwa zwischen 60 und 200° C, insbesondere etwa 80 und 170° C.

Für die Umlagerung in der Schmelze eignen sich als Katalysatoren vor allem die vorerwähnten organischen Basen, insbesondere Trialkylamine mit je 1—8 C-Atomen in den Alkylteilen; ferner Salze von Halogenwasserstoffsäuren mit Ammoniak oder organischen stickstoffhaltigen Basen, wie Trialkylaminhydrochloride und -bromide mit je 1—8 C-Atomen in den Alkylteilen, und ganz besonders

Tetraalkylammoniumhalogenide, vor allem -chloride, -bromide und -jodide, mit je 1—18 C-Atomen in den Alkylteilen.

Geeignete inerte organische Lösungsmittel sind z. B.:

— gegebenenfalls nitrierte oder halogenierte aliphatische, cycloaliphatische oder aromatische Kohlenwasserstoffe, wie n-Hexan, n-Pentan, Cyclohexan, Benzol, Toluol, Xylole, Nitrobenzol, Chloroform, Tetrachlorkohlenstoff, Trichloräthylen, 1,1,2,2-Tetrachloräthan, Nitromethan, Chlorbenzol, Dichlorbenzole und Trichlorbenzole;
— aliphatische und aromatische Nitrile, wie Alkylnitrile mit 2—5 C-Atomen, z. B. Acetonitril, Propionitril, Butyronitril, Benzonitril;
— 3-Alkoxypropionitrile mit 1 oder 2 C-Atomen im Alkoxyteil, wie 3-Methoxypropionitril und 3-Äthoxypropionitril,
— aliphatische Ketone mit bevorzugt insgesamt 3—8 C-Atomen, wie Aceton, Diäthylketon, Methylisopropylketon, Di-isopropylketon, Methyl-tert-butylketon;
— Alkyl- und Alkoxyalkylester von aliphatischen Monocarbonsäuren mit insgesamt 2—6 C-Atomen, wie Ameisensäuremethyl- und -äthylester, Essigsäuremethyl-, -äthyl-, -n-butyl- und -isobutylester sowie 1-Acetoxy-2-methoxyäthan;
— cyclische Äther, wie Tetrahydrofuran, Tetrahydropyran und Dioxan;
— Dialkyläther mit je 1—4 C-Atomen in den Alkylteilen, wie Diäthyläther, Di-n-propyläther, Di-isopropyläther;
— niedere aliphatische Alkohole, z. B. solche mit bis zu 6 C-Atomen, wie Methanol, Äthanol, Propanol, Isopropanol, Butanole und Pentanole;
— aliphatische Diole, wie Äthylenglykol und Diäthylenglykol;
— Äthylenglykol- und Diäthylenglykolmono- oder -dialkyläther mit je 1—4 C-Atomen in den Alkylteilen, wie Äthylenglykol-monomethyl- und -monoäthyläther, Diäthylenglykolmonomethyl- und -monoäthyläther, Äthylenglykoldimethyl-, -diäthyl- und -di-n-butyläther, Diäthylenglykoldiäthyl- und -di-n-butyläther;
— N,N-disubstituierte Amide von aliphatischen Monocarbonsäuren mit 1—3 C-Atomen im Säureteil, wie N,N-Dimethylformamid, N,N-Dimethylacetamid, N,N-Diäthylacetamid, N-Methyl-N-phenylacetamid und N,N-Dimethylmethoxy-acetamid;
— cyclische Amide, wie N-Methyl-2-pyrrolidon, N-Acetyl-2-pyrrolidon und N-Methyl-ε-caprolactam;
— Amide der Kohlensäure, wie Tetramethylharnstoff und Dimorpholinocarbonyl;
— Amide der phosphorigen Säure, der Phosphorsäure, der Phenylphosphonsäure oder von aliphatischen Phosphonsäuren mit 1—3 C-Atomen im Säureteil, wie Phosphorsäuretriamid, Phosphorsäuretris-(dimethylamid) (Hexametapol), Phosphorsäuretrimorpholid, Phosphorsäuretripyrrolinid, Phosphorsäure-bis(dimethylamid)-morpholid, Phosphorsäure-dimethylamid-diäthylamid-morpholid, Phosphorigsäure-tris-(di-methylamid), Tetramethyldiamid der Methanphosphonsäure;
— Amide der Schwefelsäure, von aliphatischen oder aromatischen Sulfonsäuren, wie Tetramethylsulfamid, Dimethylamid der Methansulfonsäure oder p-Toluolsulfonsäureamid;
— schwefelhaltige Lösungsmittel, wie organische Sulfone und Sulfoxide, z. B. Dimethylsulfoxid und Sulfolan.

Für die Umlagerung in Gegenwart eines sauren Katalysators verwendet man mit Vorteil polare Lösungsmittel, insbesondere niedere Alkohole, wie Methanol, Äthanol und Butanole, N,N-Dialkylamide von aliphatischen Monocarbonsäuren mit 1—3 C-Atomen im Säureteil, besonders N,N-Dimethylformamid, oder Dialkylsulfoxide, wie Dimethylsulfoxid.

In aprotischen, stark polaren Lösungsmitteln, wie den vorerwähnten N,N-disubstituierten Amiden von aliphatischen Monocarbonsäuren, cyclischen Amiden, Amiden der Kohlensäure, Amiden der phosphorigen Säure, der Phosphorsäure, der Phenylphosphonsäure oder von aliphatischen Phosphonsäuren, Amiden der Schwefelsäure, von aliphatischen oder aromatischen Sulfonsäuren, sowie Dialkylsulfoxiden, wie Dimethylsulfoxid, läuft die Reaktion auch ohne Zusatz von Base oder Säure ab. In diesen Fällen wirkt das Lösungsmittel als Katalysator.

Im allgemeinen werden jedoch bei der Umlagerung in Gegenwart eines inerten organischen Lösungsmittels als Katalysatoren bevorzugt organischen Basen mit einem pKa-Wert von über 9, insbesondere Trialkylamine mit je 1—8 C-Atomen in den Alkylteilen, wie Triäthylamin, Tri-n-butylamin und Tri-n-octylamin; ferner Halogenwasserstoffsäuren, besonders HCl, und HBr, sowie Tetraalkylammoniumhalogenide, besonders -chloride, -bromide und -jodide mit je 1—18 C-Atomen in den Alkylteilen verwendet.

Besonders bevorzugte Lösungsmittel sind aliphatische Alkohole mit 2—4 C-Atomen, Toluol, Xylole, Chlorbenzol, Dioxan, Acetonitril, 3-Methoxypropionitril, Äthylenglykoldiäthyläther und Di-isopropylketon.

Die Reaktionstemperaturen für die Umlagerung in Gegenwart eines inerten organischen Lösungsmittels liegen im allgemeinen zwischen etwa 60° und 150° C, bevorzugt zwischen etwa 80 und 130° C.

Nach beendeter Umlagerung können die Verbindungen der Formel I mit X=H und Y=Chlor auf übliche Weise isoliert und allenfalls gereinigt werden, z. B. durch Filtrieren, Destillieren und Umkristallisieren aus geeigneten organischen Lösungsmitteln, wie n-Hexan.

Die Verbindungen der Formel I und II stellen wertvolle Zwischenprodukte zur Herstellung von Schädlingsbekämpfungsmitteln dar. Sie können auch nach einer neuen, insgesamt eigenartigen Synthese in pyrethroidartige Schädlingsbekämpfungsmittel oder Vorläufer davon übergeführt werden. Derartige Verbindungen, beispielsweise solche der Formel

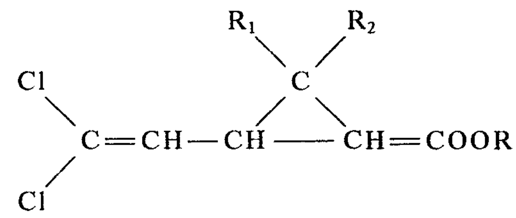

(VIII)

worin

$R_1$ und $R_2$ die unter Formel I angegebene Bedeutung haben,
R Wasserstoff, Alkyl mit 1—4 C-Atomen oder eine Gruppierung

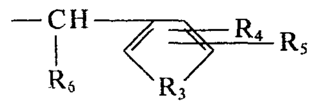

darstellt, wobei

$R_3$ —O—, —S— oder —CH=CH—,
$R_4$ Wasserstoff, Alkyl mit 1—4 C-Atomen, Benzyl, Phenoxy oder Phenylmercapto,
$R_5$ Wasserstoff oder Alkyl mit 1—4 C-Atomen und
$R_6$ Wasserstoff oder Äthinyl oder, wenn eines von $R_1$ und $R_2$ Methyl und das andere Wasserstoff oder Methyl, $R_3$—CH=CH—, $R_4$ Phenoxy und $R_5$ Wasserstoff bedeuten, auch Alkyl mit 1—5 C-Atomen darstellen,

können dadurch erhalten werden, daß man eine Verbindung der Formel I mit X=H und Y=Chlor in Gegenwart einer Base, z. B. einer Verbindung der Formel

$$M^{n+}(O^- R)_n \qquad (IX)$$

worin

M ein Alkalimetall- oder Erdalkalimetallkation und
n die Zahl 1 oder 2 bedeuten und R die unter Formel VIII angegebene Bedeutung hat,

in eine Verbindung der Formel VIII überführt.

Nach dieser neuen, erfinderischen Synthese können über die neuen Ausgangsprodukte der Formeln II und die neuen Verbindungen der Formel I Verbindungen der Formel VIII im Vergleich zu bekannten Verfahren [vgl. z. B. Farkas et al., Coll. Czech. Chem. Comm., 24, 2230 (1959) und Chem. Listy 52, 688 (1958); britische Patentschrift 1 285 350; belgische Patentschrift 850 402, deutsche Offenlegungsschriften 2 439 177, 2 539 895, 2 544 150, 2 547 510, 2 552 615, 2 554 380, 2 605 398, 2 615 159, 2 615 160, 2 616 528, 2 621 830, 2 621 832, 2 621 833, 2 621 835, 2 623 777, 2 630 981 und 2 639 777; US Patentschrift 3 961 070 und japanische Offenlegungsschriften 69 872/76 und 47 966/76] auf besonders einfache und wirtschaftliche Weise, in guten bis sehr guten Ausbeuten und mit hohen Anteilen der erwünschten, bei gewissen Anwendungen besonders wirksamen cis-Form hergestellt werden. Im Gegensatz zu vorbekannten Verfahren sind die Ausgangsprodukte leicht zugänglich, die verwendeten Reagenzien sind vergleichsweise billig und ökologisch günstig, sämtliche Reaktionsschritte können unter relativ milden Bedingungen und ohne großen apparativen Aufwand und sicherheitstechnische Maßnahmen durchgeführt werden. Die Verbindungen der Formel I und Ia weisen die für die Weiterverwendung erforderliche Oxidationsstufe auf, so daß weder Oxidationen noch Reduktionen erforderlich sind. Die einzelnen Verfahrensschritte können auch ohne Isolierung des jeweiligen Zwischenprodukts unmittelbar nacheinander durchgeführt werden, so daß sich diese Synthese sehr gut für die großtechnische Herstellung von Verbindungen der Formel VIII eignet.

Verbindungen der Formel VIII, worin R Wasserstoff oder Alkyl mit 1—4 C-Atomen darstellt, können auf an sich bekannte Weise in Wirkstoffe der Formel VIII mit R≠H oder Alkyl mit 1—4 C-Atomen übergeführt werden, oder auch in Verbindungen der Formel VIII, worin $R_6$ zusätzlich —CN bedeutet,

z. B. durch Umsetzung mit entsprechenden Halogeniden oder Alkoholen, gegebenenfalls unter vorheriger Überführung in das Säurechlorid, oder durch Umesterung vgl. z. B. die deutschen Offenlegungsschriften 2 553 991 und 2 614 648.

Verbindungen der Formel VIII mit R≠H oder Alkyl mit 1—4 C-Atomen eignen sich zur Bekämpfung von verschiedenartigsten tierischen oder pflanzlichen Schädlingen, besonders als Insektizide. Die Eigenschaften, Anwendungsgebiete und Anwendungsformen dieser Wirkstoffe sind in der Literatur beschrieben [vgl. Nature, 246, 169—70 (1973); Nature, 248, 710—11 (1974); Proceedings 7th British Insecticide and Fungicide Conference, 721—728 (1973); Proceedings 8th British Insecticide and Fungicide Conference, 373—78 (1975); J. Agr. Food Chem., 23, 115 (1975); US Patentschrift 3 961 070; deutsche Offenlegungsschriften 2 553 991, 2 439 177, 2 326 077 und 2 614 648].

## Beispiel 1

166,4 g (0,8 Mol) 2,4,4-Trichlorbut-3-encarbonsäurechlorid in 1,6 Liter n-Hexan werden mit Isobutylen gesättigt. Unter Rühren und Einleiten eines schwachen Stroms von Isobutylen werden bei Raumtemperatur (20—25°C) während 7 Stunden 80,8 g (0,8 Mol) Triäthylamin zugetropft. Nach einer Stunde Nachrühren wird filtriert. Das Filtrat wird zuerst mit verdünnter Natronlauge, dann mit verdünnter Salzsäure und schließlich mit Wasser gewaschen, über Magnesiumsulfat getrocknet und eingedampft. Der Rückstand wird destilliert. Man erhält 2-Chlor-2-(2′,2′-dichlorvinyl)-3,3-dimethylcyclobutanon vom Sdp. 54°C/13,33 Pa. (0,1 mm Hg) als farblose Flüssigkeit.

IR-Spektrum ($CHCl_3$) in $cm^{-1}$: 1800 (CO), 1620 (C=C).

NMR-Spektrum (100 MHz, $CDCl_3$) in ppm: 1,38 (s, 3H, $CH_3$); 1,53 (s, 3H, $CH_3$); 2,77—3,29 (m, 2H, $CH_2$); 6,17 (s, Is, C=CH).

Elementaranalyse für $C_8H_9Cl_3O$ (Molgewicht 227,52):

| | | | |
|---|---|---|---|
| berechnet | C 42,23 | H 3,99 | Cl 46,75% |
| gefunden | C 41,98 | H 3,85 | Cl 47,02%. |

## Beispiel 2

15,6 g (86,5 mMol) des gemäß Beispiel 2 hergestellten 2-Chlor-2-(2′,2′-dichlorvinyl)-3,3-dimethylcyclobutanons und 5 g Tetrabutylammoniumchlorid werden zusammen während 4 Stunden bei 90°C gerührt. Die abgekühlte Schmelze wird in n-Hexan aufgenommen und klarfiltriert. Das eingedampfte Filtrat wird auf Kieselgel chromatographiert (Eluierung mit Cyclohexan/Toluol im Volumenverhältnis 1 : 1). Man erhält 2-(2′,2′-Dichlorvinyl)-3,3-dimethyl-4-chlorcyclobutanon als farbloses Öl.

IR-Spektrum ($CDCl_3$) in $cm^{-1}$: 1780 (CO), 1670 (C=C).

NMR-Spektrum ($CDCl_3$, 100 MHz) in ppm: 1,10 (s, 3H, $CH_3$); 1,60 (s, 3H, $CH_3$); 3,98 (dd, J=8Hz und 2 Hz, 1H, H—$C_2$); 4,73 (d, J=2Hz, 1H, H—$C_4$); 5,80 (d, J=8 Hz, 1H, CH=$CCl_2$).

Das obige 2-(2′,2′-Dichlorvinyl)-3,3-dimethyl-4-chlorcyclobutanon kann wie folgt in Verbindungen der Formel VIII übergeführt werden:

4,55 g (20 mMol) 2-(2′,2′-Dichlorvinyl)-3,3-dimethyl-4-chlorcyclobutanon werden mit einer Lösung von 0,88 g (22 mMol) festem Natriumhydroxid in 40 ml Wasser versetzt und bei Raumtemperatur während 25 Stunden gerührt. Die nun klare Lösung wird mit Diäthyläther gewaschen, mit halbkonzentrierter Salzsäure sauer gestellt und mit Diäthyläther extrahiert. Der Extrakt wird mit Wasser gewaschen, über Magnesiumsulfat getrocknet und eingedampft. Der Rückstand wird aus n-Hexan kristallisiert. Man erhält die bekannte cis-2-(2′,2′-Dichlorvinyl)-3,3-dimethylcyclopropancarbonsäure; Smp. 86—87°C.

10 g (0,047 Mol) cis-2-(2′,2′-Dichlorvinyl)-3,3-dimethylcyclopropancarbonsäure werden in 100 ml Benzol mit 12,1 ml (0,141 Mol) Oxalylchlorid 24 Stunden bei Raumtemperatur gerührt. Nach dem Eindampfen der Reaktionslösung wird der braune Rückstand unter vermindertem Druck destilliert. Man erhält 9,1 g einer klaren Flüssigkeit; Sdp. 50°C/5,32 Pa (0,04 mm Hg). 3,0 g dieser klaren Flüssigkeit werden in 30 ml Toluol gelöst und mit 2 ml Pyridin versetzt. Bei Raumtemperatur werden dazu 2,9 g α-Cyano-m-phenoxybenzylalkohol in 20 ml Toluol getropft, und das Reaktionsgemisch wird anschließend während 16 Stunden bei Raumtemperatur weitergerührt. Das Reaktionsgemisch wird zuerst mit Wasser, dann mit gesättigter Natriumhydrogencarbonatlösung und anschließend mit Sole gewaschen, über Magnesiumsulfat getrocknet und eingedampft. Der Rückstand wird über Kieselgel chromatographiert (Eluierung mit Diäthyläther/n-Hexan 1 : 2). Man erhält reinen cis-2-(2′,2′-Dichlorvi-

nyl)-3,3-dimethylcyclopropancarbonsäure-α-cyan-m-phenoxybenzylester als Diastereoisomerengemisch.

NMR-Spektrum (60 MHz, $CDCl_3$) in ppm: 1,20—1,43 (m, 6H, 2x $CH_2$); 1,67—2,35 (m, 2H 2x CH); 6,25 (d, J = 9 Hz, 1H, $CH=CCl_2$); 6,40 und 6,45 (je ls, je 0,5 H, CH—CN); 6,98—7,65 (m, 9 H).

**Patentansprüche**

1. Eine Verbindung der Formel

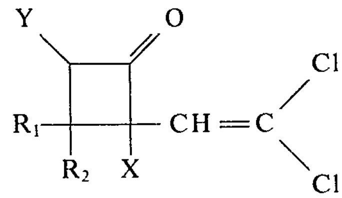

worin

eines von $R_1$ und $R_2$ Methyl und das andere Wasserstoff oder Methyl oder
$R_1$ und $R_2$ zusammen Alkylen mit 2—4 C-Atomen darstellen,
X Chlor und Y Wasserstoff oder
X Wasserstoff und Y Chlor bedeuten.

2. Eine Verbindung gemäß Anspruch 1, worin $R_1$ und $R_2$ je Methyl oder zusammen Äthylen darstellen.
3. Eine Verbindung gemäß Anspruch 1, worin $R_1$ und $R_2$ je Methyl bedeuten.
4. Ein Verfahren zur Herstellung einer Verbindung gemäß Anspruch 1, dadurch gekennzeichnet, daß man eine Verbindung der Formel

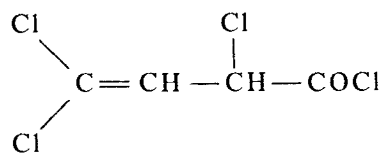

in Gegenwart einer organischen Base besonders Pyridin oder ein Trialkylamin mit je 1—4 C-Atomen in den Alkylteilen, insbesondere Triäthylamin, gegebenenfalls in Gegenwart eines inerten organischen Lösungsmittels, besonders n-Hexan oder Cyclohexan mit einer Verbindung der Formel

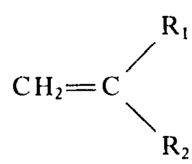

zu einer Verbindung der Formel

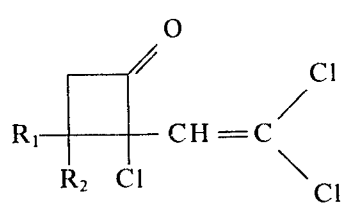

umsetzt, wobei $R_1$ und $R_2$ die unter Formel I angegebene Bedeutung haben, und diese Verbindung in Gegenwart eines Katalysators besonders einer Halogenwasserstoffsäure oder einer organischen Base insbesondere Trialkylamin mit 1—8 C-Atomen in den Alkylteilen oder Tetraalkylammoniumhalogenid mit 1—18 C-Atomen in den Alkylteilen gegebenenfalls in Gegenwart eines inerten organischen Lösungsmittels, bei einer Temperatur zwischen 80 und 130°C gegebenenfalls zu einer Verbindung gemäß Anspruch 1 umlagert, worin X Wasserstoff und Y Chlor bedeutet.

**Claims**

1. A compound of the formula

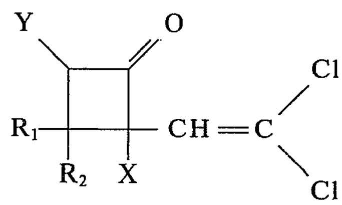

wherein

one of $R_1$ and $R_2$ is methyl and the other is hydrogen or methyl, or
$R_1$ and $R_2$ together are alkylene having 2—4 C atoms,
X ist chlorine and Y is hydrogen, or
X is hydrogen and Y is chlorine.

2. A compound according to Claim 1, wherein $R_1$ and $R_2$ are each methyl, or together are ethylene.
3. A compound according to Claim 1, wherein $R_1$ and $R_2$ are each methyl.
4. A process for producing a compound according to Claim 1, wich process comprises reacting a compound of the formula

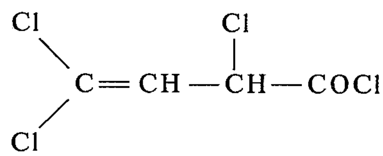

in the presence of an organic base, particularly pyridine or a trialkylamine having 1—4 C atoms in each of the alkyl moieties, especially triethylamine, optionally in the presence of an inert organic solvent, particularly n-hexane or cyclohexane, with a compound of the formula

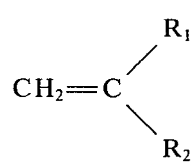

to give a compound of the formula

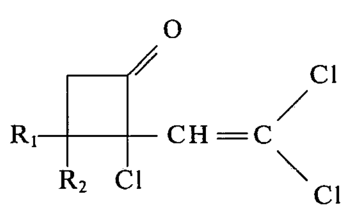

wherein $R_1$ and $R_2$ have the meanings defined under the formula I; and optionally rearranging this compound, in the presence of a catalyst, particularly a hydrohalic acid or an organic base, especially trialkylamine, having 1—8 C atoms in each of the alkyl moieties or tetraalkylammonium halide having 1—18 C atoms in each of the alkyl moieties, optionally in the presence of an inert organic solvent, at a temperature of between 80 and 130° C, to a compound according to Claim 1 wherein X is hydrogen and Y is chlorine.

**Revendications**

1. Composé de formule:

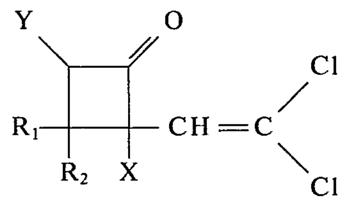

où l'un des radicaux $R_1$ et $R_2$ représente un méthyle et l'autre un hydrogène ou un méthyle, ou bien où $R_1$ et $R_2$ représentent ensemble un alcoylène ayant de 2 à 4 atomes de carbone, X un chlore et Y un hydrogène ou X un hydrogène et Y un chlore.

2. Composé selon la revendication 1, où $R_1$ et $R_2$ représentent chacun un méthyle ou ensemble un éthylène.

3. Composé selon la revendication 1, où $R_1$ et $R_2$ représentent chacun un méthyle.

4. Procédé de préparation d'un composé selon la revendication 1, caractérisé en ce qu'on fait réagir un composé de formule:

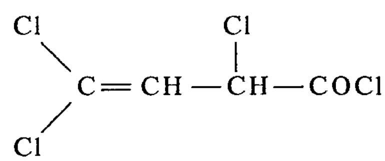

en présence d'une base organique avec un composé de formule:

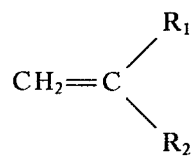

pour donner un composé de formule:

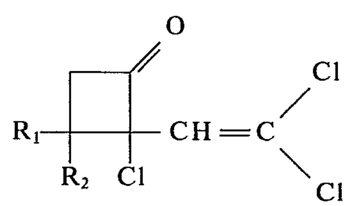

où $R_1$ et $R_2$ ont la signification donnée sous la formule I, et en ce qu'on transpose ce composé en présence d'un catalyseur, en particulier d'un acide halohydrique ou d'une base organique, en particulier une trialcoylamine ayant de 1 à 8 atomes de carbone dans la partie alcoyle ou d'un halogénure de tétraalcoylammonium ayant de 1 à 18 atomes de carbone dans la partie alcoyle, éventuellement en présence d'un solvant organique inerte, à une température comprise entre 80 et 130° C, le cas échéant en un composé selon la revendication 1, où X représente un hydrogène et Y un chlore.